# EUROPEAN PATENT APPLICATION

(11) **EP 2 351 543 A1**
(43) Date of publication of application: **03.08.2011**
(21) Application number: 09823359.6
(22) Date of filing: 29.01.2009
(51) Int. Cl.: A61F 5/56

(54) **TONGUE POSITION CONTROLLER**

(30) Priority: 30.10.2008 JP 2008280205
(71) Applicant: Tsuiki, Satoru, Tokyo 181-0001 (JP); Minamino, Osamu, Saitama 362-0805 (JP)
(72) Inventor: Tsuiki, Satoru, Tokyo 181-0001 (JP); Minamino, Osamu, Saitama 362-0805 (JP)
(74) Representative: Leeming, John Gerard
(86) International application number: PCT/JP2009/051427
(87) International publication number: WO 2010/050246

(57) **Abstract**

Provided is a device which effectively improves obstructive sleep apnea syndrome (OSAS), which places a small psychosomatic burden on the patient, and which is unlikely to cause a decrease in compliance or adverse side effects.

The tongue position controller of the present invention includes: a mounting portion composed of a splint applied to a lower jaw dentition, and a capsule which is arranged on an inner curved portion of an upper surface of the splint, which is composed so as to cover at least a portion of a tongue when the splint is applied to the lower jaw dentition, and which has a through hole positioned at a tip of the tongue when the splint is applied; a pressure generating device that generates a negative pressure; and a tube that connects the through hole and the pressure generating device.

## Description

### BACKGROUND ART

The present invention relates to a tongue position controller that is used while sleeping for the treatment of snoring and obstructive sleep apnea syndrome and the like.

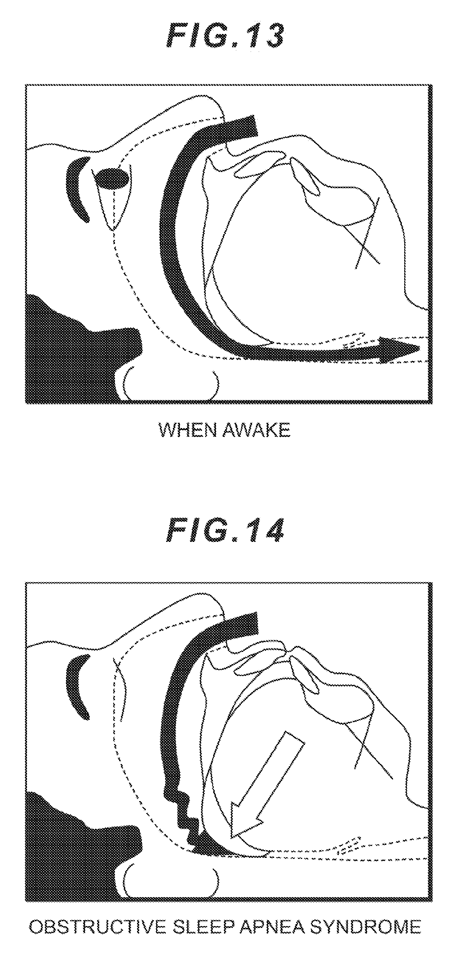

Obstructive sleep apnea syndrome (OSAS) is a disease that causes apnea due to obstruction of the upper airway while sleeping. Recently, two major factors have been identified to be responsible for the onset of OSAS. The first factor is a decrease in the activity of upper airway dilator muscles (including genioglossus muscle) that is observed at the stage from waking to sleep. Although the upper airway is open when awake (indicated by the black arrow in FIG. 13), the tongue moves to the direction of the throat and pushes the soft palate backward because of the sleep-induced decrease in the activity of upper airway dilator muscles (indicated by the white arrow in FIG. 14). As a result, the portion of the upper airway posterior to the soft palate (the velopharynx) becomes particularly narrow, and patency of airway decreases (indicated the black arrow in FIG. 14). In fact, the primary site of upper airway obstruction in OSAS has been identified as the velopharynx. This decrease in the activity of upper airway dilator muscle is a physiological phenomenon that is also observed in healthy persons (non-OSAS patients), and the presence of this phenomenon alone does not contribute to the onset of OSAS. The second factor currently proposed is an anatomical abnormality in upper airway morphology. For example, in the case the upper airway is inherently constricted when waking up due to excessive intraoral soft tissue caused by obesity, upper airway patency is thought to decrease considerably resulting in the occurrence of OSAS due to even slight narrowing of the upper airway caused by falling asleep.

The prevalence of OSAS in adults is high at about 2 to 4% in general population, and since OSAS increases the risk of traffic accidents and industrial accidents due to the excessive daytime sleepiness and is a causative factor of metabolic syndrome and cerebrovascular diseases, the importance of the development of an effective treatment method is widely recognized.

Conventional OSAS therapy is broadly divided into surgical treatment and conservative treatment. Surgical procedures such as uvulopalatopharyngoplasty (UPPP) and maxillomandibular advancement (MMA) provide a radical cure of OSAS if successful. Conversely, since these procedures can cause problems of pronunciation and swallowing following surgery and/or cause recurrence of OSAS, and since the surgery is irreversible, the number of patients who select surgical treatment is limited.

On the other hand, conservative therapy, which does not rely on surgical procedures, includes nasal continuous positive airway pressure (nCPAP), which is the first treatment of choice for OSAS, and the use of an oral appliance (mouthpiece), which is currently the second treatment of choice (see, for example, Non-Patent Document 1). nCPAP prevents upper airway obstruction by maintaining the airway pressure at positive with the use of positive pressure applied from a mask attached to the nose. In addition, an oral appliance is a device that advances the lower jaw or tongue to maintain the upper airway enlarged. Oral appliances are broadly classified into two types: mandibular advancement devices (MADs) and tongue retaining devices (TRD) (see, for example, Non-Patent Document 2). Although both nCPAP and oral appliances are established treatment methods, these methods do not bring about a radical cure for OSAS, and patients must use these devices for a long period of time,

Although nCPAP is associated with a high OSAS improvement rate, it also has a low rate of compliance (probability of being able to be used as instructed) and numerous patients discontinue treatment due to the nasal symptoms caused by the positive airway pressure and the discomfort resulting from wearing the mask. In addition, when positive airway pressure is applied in the upper airway using nCPAP, upper airway muscle activity is inhibited (see, for example, Non-Patent Document 3). Namely, although nCPAP unquestionably results in improvement of OSAS, it also lowers the defensive functions against OSAS inherently possessed by patients.

In addition, if nCPAP is used in pediatric OSAS patients, growth of the maxillary bone is inhibited as a result of the external force applied to the maxillary bone by the mask attached to the nose, and this has been reported to cause mid-face deformation and changes in occlusion in pediatric OSAS patients (see, for example, Non-Patent Document 4). Occlusal changes similar to those observed in children have also been reported to occur in adult OSAS patients caused by external force of the mask in long-term nCPAP user (see, for example, Non-Patent Document 5).

In contrast, oral appliances are convenient, inexpensive, easy to use and can also be applied to patients who have discontinued nCPAP therapy. Conversely, they have been indicated to not be as effective as nCPAP (see, for example, Non-Patent Document 6). Although MADs are more effective in improving OSAS than TRD, since they cause the lower jaw to be immobilized in an advanced jaw position, the load on the teeth and temporomandibular joint is large, and there is a high likelihood of causing adverse side effects such as dislocation of teeth and discomfort and pain in the temporomandibular joint resulting from long-term use (see, for example, Non-Patent Document 7). Consequently, there is a considerable psychosomatic burden on patients resulting from the use of these devices.

In addition, in the case of using MAD in pediatric OSAS patients, the MAD acts as an orthodontic appliance that promotes growth of the lower jaw due to mandibular advancement resulting from use of this device (see, for example, Non-Patent Document 8). Consequently, although symptoms of OSAS can be expected to be improved, there is also concern over the occurrence of anterior crossbite, Consequently, in the case of using MAD in pediatric OSAS patients, MAD use is limited to patients having a small lower jaw.

Although TRD is also a type of oral appliance, its design and treatment principle differ from those of MAD. Although the use of a mouthpiece integrated with the dentitions of the upper and lower jaws is similar to the principle employed by MAD, TRD causes dilation of the airway posterior to the tongue by suctioning and maintaining the tongue in an advanced position through a socket provided in the anterior portion thereof. However, since the mouthpiece is integrated with the upper and lower jaws, the position of the lower jaw cannot be changed in the same manner as MAD. Although it is normal for the lower jaw to descend posteriorly to create an jaw opening due to the gravity and decreased activity of the jaw closing muscles while asleep (see, for example, Non-Patent Document 9), when a TRD is worn, this physiological positional change is prevented. As a result, since a load acts on the teeth of the upper and lower jaws through the mouthpiece, there are harmful effects on the teeth attributable to long-term use in the same manner as MAD (see, for example, Non-Patent Document 10). There is also a considerable psychosomatic burden on patients caused by immobilization of the lower jaw and advancing the tongue.

Moreover, since nCPAP and TRD prevent mouth breathing, they cannot be used by patients who have difficulty in breathing through the nose. It is thought that humans inherently breathe through the nose and that this is the physiological mode of respiration (see, for example, Non-Patent Documents 11 and 12). In consideration of this point, it is reasonable to assume that nGPAP and TRD basically employ a design that is premised on breathing through the nose at the time of use. However, in cases in which the nasal cavity has become completely obstructed for whatever reason, route of respiration is maintained by switching from nose breathing to mouth breathing. Moreover, even in cases in which nasal obstruction has occurred to an extent that does not result in complete obstruction or under certain physiological conditions (such as in cases of increased ventilation rate), humans begin partial mouth breathing (see, for example, Non-Patent Documents 11 and 13). In addition, even among normal, healthy adults, there are persons who have been reported to breathe through the both the nose and mouth in addition to persons who breathe through the nose (see, for example, Non-Patent Document 14).

The mouth serves as an important respiration route among OSAS patients engaged in partial mouth breathing. In addition, OSAS patients are frequently complicated with nasal occlusion accompanying allergic rhinitis and other similar conditions (see, for example, Non-Patent Document 13). nCPAP compliance in these patients decreases considerably. If a patient wearing an nCPAP device attempted to breathe through the mouth, positive pressure in the upper airway would be released and air would leak out through the mouth thereby rendering nCPAP therapy ineffective.

In addition, among these oral appliances, although MAD may be provided with an air hole between upper and lower jaw splints, compliance decreases in cases in which it is structurally impossible to secure an adequate size for the air hole.

In addition, the inherent objective of TRD is to promote nose breathing by preventing mouth breathing in addition to improving OSAS (see Patent Document 1). TRD prevents mouth breathing by virtue of their structure that provides upper and lower jaw splints and a socket positioned anterior to the tongue at an intermediate position thereto.

Moreover, a method and device for treating OSAS has been proposed that generates negative pressure within the oral cavity of a patient (see, for example, Patent Document 2). The generation of negative pressure in the oral cavity causes the patient's soft palate and surrounding soft tissue to be drawn into the oral cavity and the tongue to drawn towards the hard palate, thereby maintaining the patient's upper airway in an open state. However, the burden on the patient caused by this non-physiological state of creating negative pressure throughout the oral cavity is predicted to be considerable, thereby leaving the problem of compliance unresolved. In addition, since a mouthpiece integrated with the upper and lower jaws is worn, obstruction of physiological positional changes of the upper jaw, being unable to breathe through the mouth, and concerns over the occurrence of adverse side effects on the teeth resulting from the wearing the mouthpiece for a long period of time are the same as in the case of MAD and TRD.

Similarly, a method has also been proposed for preventing the tongue from descending towards the upper airway while asleep by holding the patient's tongue against the palate using a suction cup and drawing negative pressure within the suction cup (see, for example, Patent Document 3). However, this method does not provide a way to deal with the sense of a foreign object present in the oral cavity caused by adhesion of the suction cup to the surface of the tongue, does not take into consideration physiological changes in the position of the lower jaw while asleep, and does not provide a way to deal with changes in the position of the lower jaw, and the safety of this method is unctear.
[Patent Document 1] U.S. Patent No. 4169473
[Patent Document 2] Japanese Patent Application Laid-open No. 2007-319680
[Patent Document 3] Japanese Patent Application Laid-open No. 2008-183388
[Non-Patent Document 1] Tsuiki S, Inoue Y. Clinical Sleep Medicine (in Japanese) 1st edition, 1st printing; Nippon Rinsho Suppl 66, Supplement 2, 2008; pp 182-186.
[Non-Patent Document 2] Cartwright RD, Samelson CF. JAMA 1982; 248: 705-709.
[Non-Patent Document 3] Strohl KP, Redline S. Am Rev Respir Dis 1986; 134: 555-558.
[Non-Patent Document 4] Li KK, et al. Chest 2000; 117: 916-918.
[Non-Patent Document 5] Lowe AA. Efficacy of oral appliance therapy as an adjunct to nCPAP. American Academy of Dental Sleep Medicine 17th Annual Meeting, Baltimore, June 6-8, 2008 (abstracts of presentations).
[Non-Patent Document 6] Ferguson KA, et al. Sleep 2006; 29: 244-262.
[Non-Patent Document 7] Almeida FR, et al. J Clin Sleep Med 2005; 2:143-152.
[Non-Patent Document 8] Graber TM, In Graber TM, Varnarsdall JrRL, Eds. Orthodontics: Current Principles and Techniques, 2nd ed. St. Louis, Missouri, Mosby, 1994, pp 383-436.
[Non-Patent Document 9] Miyamoto K, et al. Arch Oral Biol 1999; 44: 657-664.
[Non-Patent Document 10] Chen H, et al. Sleep Breath 2008; 12: 169-178.
[Non-Patent Document 11] Hoffstein V. Sleep Breath 2007; 11: 1-22.
[Non-Patent Document 12] Robinson RW, Zwillich CW. In Kryger MH, Roth T, Dement WC, Eds. Principles of Practice of Sleep Medicine, 3rd ed. Philadelphia, WB Saunders, 2000; pp 797-812.
[Non-Patent Document 13] Chiba S. Sleep Breathing Disorders Update 2006 (in Japanese), Inoue K, Yamashiro Y, Eds. 1st edition, 1st punting, Nihon Hyoronsha, 2006; pp 53-60.
[Non-Patent Document 14] Ninimaa V et al. Respir Physiol 1981; 43: 69-75.

### SUMMARY

An object of the present invention is to provide a device that effectively improves OSAS, places a small psychosomatic burden on the patient, and is unlikely to cause a decrease in compliance or adverse side effects.

As a result of conducting extensive studies with the foregoing in view, the inventors of the present invention found that preventing obstruction of the posterior portion of the soft palate is effective for improving OSAS, that obstruction of the posterior of the soft palate by preventing settling of the tongue can be prevented directly by suctioning the tongue using a mounting portion composed of a splint independently applied to the lower jaw and a capsule without using a mouthpiece integrated with the upper and lower jaws, and physiological movement and positional changes of the lower jaw are not impaired while allowing mouth breathing by using a splint independently applied to the lower jaw, thereby leading to completion of the present invention.

Namely, the present invention relates to:
[1] a tongue position controller including: a mounting portion composed of a splint applied to a lower jaw dentition, and a capsule which is arranged on an inner curved portion of an upper surface of the splint, which is composed so as to cover at least a portion of a tongue when the splint is applied to the lower jaw dentition, and which has a through hole positioned at a tip of the tongue when the splint is applied; a pressure generating device that generates a negative pressure; and a tube that connects the through hole and the pressure generating device;
[2] the tongue position controller according to [1] above, further including a pressure adjustment mechanism that automatically adjusts negative pressure generated by the pressure generating device;
[3] the tongue position controller according to [2] above, wherein the pressure adjustment mechanism includes a pressure sensor that measures negative pressure in the tube, and a valve which is provided in a path connecting the tube with outside air, and an opening and closing of which is controlled according to a measured value of the pressure sensor;
[4] the tongue position controller according to any one of [1] to [3] above, wherein the splint and the capsule are formed from a thermoplastic or thermosetting dental material;
[5] the tongue position controller according to any one of [1] to [4] above, wherein the capsule has a bilayer structure in which a periphery is closed, a plurality of fine through holes are provided in one layer that contacts the tongue, and a through hole to which the tube is connected is provided in the other layer;
[6] the tongue position controller according to [5] above, wherein each layer of the bilayer structure employs a removable configuration;
[7] the tongue position controller according to any one of [1] to [6] above, wherein a gas-liquid separator is arranged between the through hole of the capsule and the pressure generating device, and the tube is composed of a first tube that connects the through hole of the capsule and the gas-liquid separator for discharging suctioned material inside the gas-liquid separator, and a second tube that connects a gaseous phase portion of the gas-liquid separator and the pressure generating device;
[8] the tongue position controller according to any one of [1] to [7] above, further including a face mask which holds the mounting portion, and which includes a forehead retaining portion that contacts a forehead, a lower jaw retaining portion that contacts a lower jaw, and a wire that connects and immobilizes the forehead retaining portion, the lower jaw retaining portion and the mounting portion:
[9] a control method of the tongue position controller according to [3] above, which includes the steps of: comparing a measured value of the pressure sensor with a preset pressure, and opening the valve in a case that the measured value has decreased to or below the preset pressure; and
[10] the control method according to [9] above, which further includes the steps of: comparing a measured value of the pressure sensor with a preset pressure with the valve open, and closing the valve in a case that the measured value has increased to or above the preset pressure.

Since the tongue position controller according to the present invention acts directly and reliably on the tongue, it is able to effectively improve OSAS by preventing upper airway obstruction of the posterior portion of the soft palate that causes a positional change in the tongue that is considered to be most intimately involved in the onset of OSAS.

Moreover, since the tongue position controller according to the present invention uses a splint that is independently applied to the lower jaw, it solves problems associated with conventional oral appliances employing a structure that is integrated with the upper and lower jaws such as preventing mouth breathing and obstructing physiological movement of the lower jaw. Thus, it can be preferably used even by patients who have a problem with nose breathing, there is no discomfort associated with immobilization of the lower jaw, and there is little burden on the nasal cavity or maxillofacial system. This tongue position controller also facilitates accommodation of regressive changes in various maxillofacial functions accompanying aging. In addition, it also solves problems associated with nCPAP such as discomfort caused by positive pressure or wearing a mask and inhibition of activity of upper airway dilator muscles,

In addition, by virtue of its structure, this tongue position controller does not induce growth inhibition of the maxillary bone or accelerated growth of the lower jaw even used in pediatric patients.

Moreover, since it is not necessary to advance the tongue, the psychosomatic burden on patients is reduced leading to expectations of greater compliance.

In addition to OSAS, the present invention is also able to improve diseases that resemble OSAS but do not involve upper airway obstruction such as upper airway resistance syndrome and snoring,

In addition, the tongue position controller according to the present invention can also be used to improve upper airway patency in cases of neurological diseases associated with respiratory abnormalities caused by changes in tongue position.

### DESCRIPTION OF THE DRAWINGS

FIG. 1 is an explanatory drawing showing an overview of a mounting portion of a tongue position controller according to the present invention;
FIG. 2A is an overhead view of a mounting portion of a tongue position controller according to the present invention, FIG. 2B is a lateral view of a mounting portion of a tongue position controller according to the present invention, and FIG. 2C is a perspective view of a mounting portion as viewed from the opposite side of a through hole;
FIG. 3 is a cross-sectional view of a cross-section taken along line III-III in FIG. 2A;
FIG. 4 consists of schematic diagrams showing states in which the tongue position controller according to the present invention is mounted in the mouth;
FIG. 5 is a cross-sectional view of an embodiment of the tongue position controller according to the present invention;
FIG. 6 consists of explanatory drawings showing overviews of a tongue position controller employing a bilayer structure for a capsule;
FIG. 7 is a cross-sectional view of a tongue position controller employing a bilayer structure for a capsule;
FIG. 8 is a schematic diagram showing an embodiment of a tongue position controller employing a bilayer structure for a capsule;
FIG. 9 is a schematic diagram showing an embodiment of bilayer connecting portion of a tongue position controller employing a bilayer structure for a capsule;
FIG. 10 is a schematic diagram showing a configuration in which a gas-liquid separator is arranged in the tongue position controller according to the present invention;
FIG. 11 consists of schematic diagrams showing an embodiment of a face mask used with the tongue position controller according to the present invention;
FIG. 12 is an overhead view showing a connecting portion of the facemask shown in FIG. 11 that connects to a capsule;
FIG. 13 is an explanatory drawing showing the prior art; and,
FIG. 14 is an explanatory drawing showing the prior art.

### DETAILED DESCRIPTION

The following provides an explanation of preferred embodiments of the present invention with reference to the drawings.

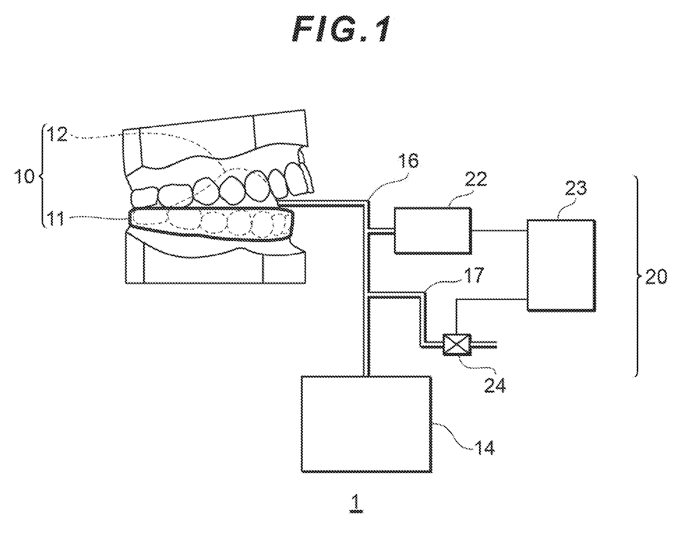

FIG. 1 is an explanatory drawing showing an overview of a tongue position controller according to the present invention.

As shown in the drawing, the tongue position controller according to the present invention is provided with a mounting portion 10 composed of a splint 11 and a capsule 12, a pressure generating device 14 such as a suction pump that generates negative pressure, and a tube 16 that connects the mounting portion 10 and the pressure generating device 14. A through hole 13 to which the tube 16 is connected is provided in the capsule 12.

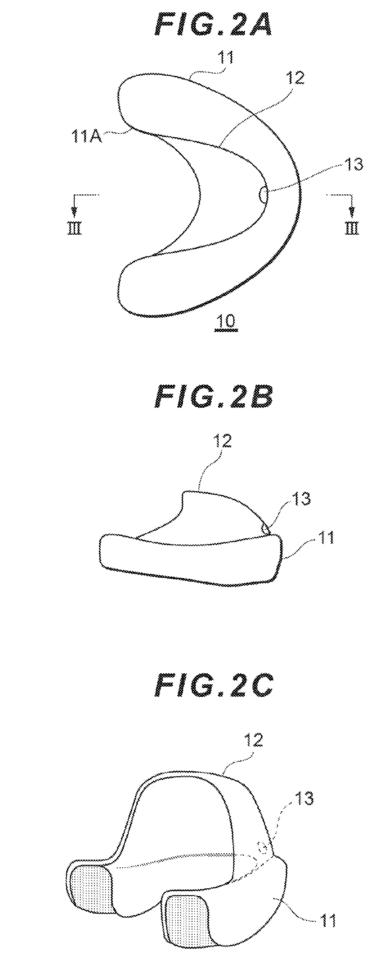

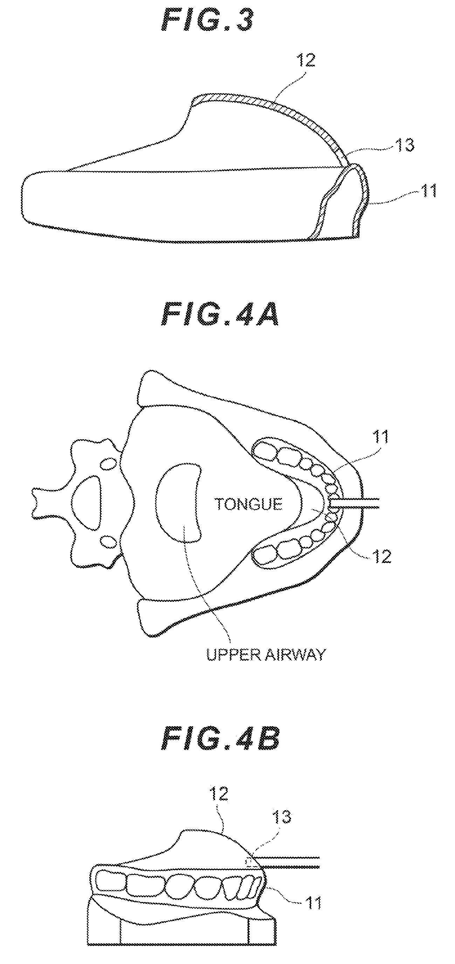

FIG. 2A shows an overhead view of the mounting portion 10 and FIG. 2B shows a side view, while FIG. 2C shows a perspective view of the mounting portion 10 as viewed from the opposite side of the through hole 13. In addition, FIG. 3 shows a cross-sectional view of a cross-section taken along line III-III in FIG. 2A.

The splint 11 is applied to at least a portion of the lower jaw dentition, and although it may be of any shape provided it fulfills the role of fixing the mounting portion within the oral cavity, it is preferably of a shape that covers the lower jaw dentition and dental alveoli. A commercially available splint may be used for the sprint 11, or can be fabricated in accordance with a method used in the field of orthodontics. For example, of a mold of the dentition can be fabricated by making an impression so as to match the dentition of the patient, and a splint can be molded by pressing a thermoplastic or thermosetting resin and the like into the mold. The material of the splint 11 may be metal or plastic and the like, there are no particular limitations thereon, that have elasticity or that not having elasticity may be used, and for example, a thermoplastic dental material is used preferably. In particular, an elastic material such as a silicon-based material or polyolefin having superior safety and feel when applied is preferable.

As shown in FIGS. 2A to 2C, the capsule 12 is arranged in a dome shape on the inner curved portion of the upper surface of the splint 11, and is composed so as to cover at least a portion of the tongue when the splint 11 is applied to the lower jaw dentition. Although there are no particular limitations on the shape of the capsule 12 provided it is able to maintain suction on the tongue when suction is drawn from the through hole 13 when the mounting portion 10 is applied, it preferably is of a shape that surrounds the top and front of the back of the tongue when applied, and covers the posterior portion of the tongue (for example, the region extending from the first to second molars).

Although the basic structure of the capsule 12 is as shown in the drawings, the shape thereof can be changed according to the patient to more easily maintain suction on the tongue. In the case of shaping to accommodate a specific patient, for example, a combined impression can be obtained of the back of the tongue and occlusal plane of the lower jaw dentition, and the shape of the capsule 12 can be designed by evaluating the relative size and position of the tongue when at rest relative to the lower jaw dental arch. At that time, a tray for obtaining a combined impression of the back of the tongue and the occlusal plane of the lower jaw dentition may be fabricated by modifying the outer surface of an individual maxillary tray. In addition, the capsule 12 can also be designed by evaluating the relative size and position of the tongue by observing the oral cavity on the mold in a state in which the dentition molds of the upper and lower jaws are occluded and evaluating the oral cavity with the molt

FIG. 4 shows the mounting portion applied inside the oral cavity. As shown in FIGS. 4A and 4B, the splint 11 is applied to the lower jaw dentition, and as a result, the capsule 12 covers a portion of the tongue.

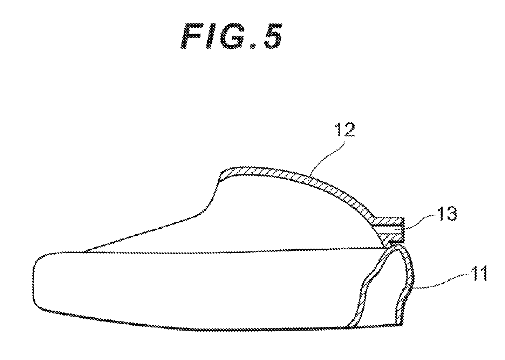

The through hole 13 for connecting a tube is provided in front of the capsule 12, namely on the opposite side from the oral cavity. The through hole 13 can be formed in accordance with a method known among persons with ordinary skill in the art, and the position thereof can be suitably changed provided the effect of suctioning the tongue is obtained. The through hole 13 may be formed as a simple through hole as shown in FIGS. 2 and 3, or may be composed to facilitate connection with the tube 16 by causing the periphery of the through hole to protrude in a cylindrical shape as shown in FIG. 5.

Although the material of the capsule 12 may be metal or plastic and the like, and there are no particular limitations thereon, a thermoplastic dental material is used preferably in the same manner as the splint 11. In particular, an elastic material such as a silicon-based material or polyolefin having superior safety and feel when applied is preferable.

The splint 11 and the capsule 12 may be attached after each is molded, or may be integrally molded. Moreover, from the viewpoint of sanitary hygiene and the like, the mounting portion 10 is preferably fabricated from an inexpensive material for disposable use. In addition, from the viewports of sanitary hygiene and environmental considerations, the mounting portion 10 and the capsule 12 are preferably removable and able to be cleaned using a commercially available dental cleaner to allow repeated use.

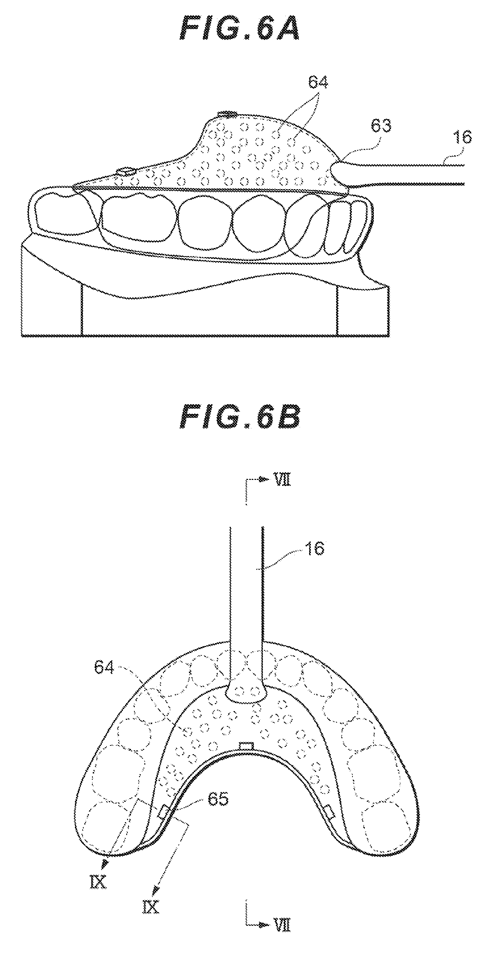

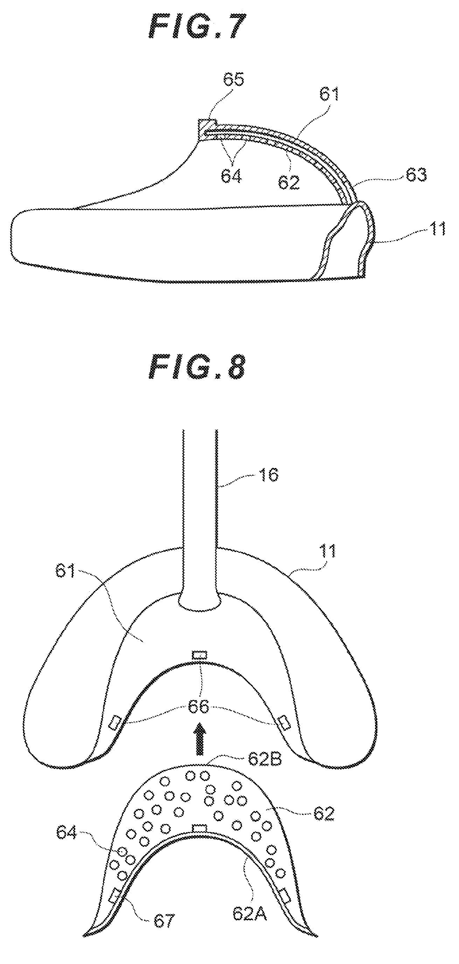

In addition, as shown in FIGS 6 to 9, the capsule 12 according to the present invention preferably has a bilayer structure having a closed periphery, a plurality of fine through holes 64 are provided in a layer 62 on the side that contacts the tongue, and a through hole 63 for connecting a tube is provided in the other layer 61. FIG. 7 is a cross-sectional view taken along line VII-VII of FIG. 6B. A space of about 1 to 4 mm, and preferably about 2 mm, is provided between the layer 61 and the layer 62.

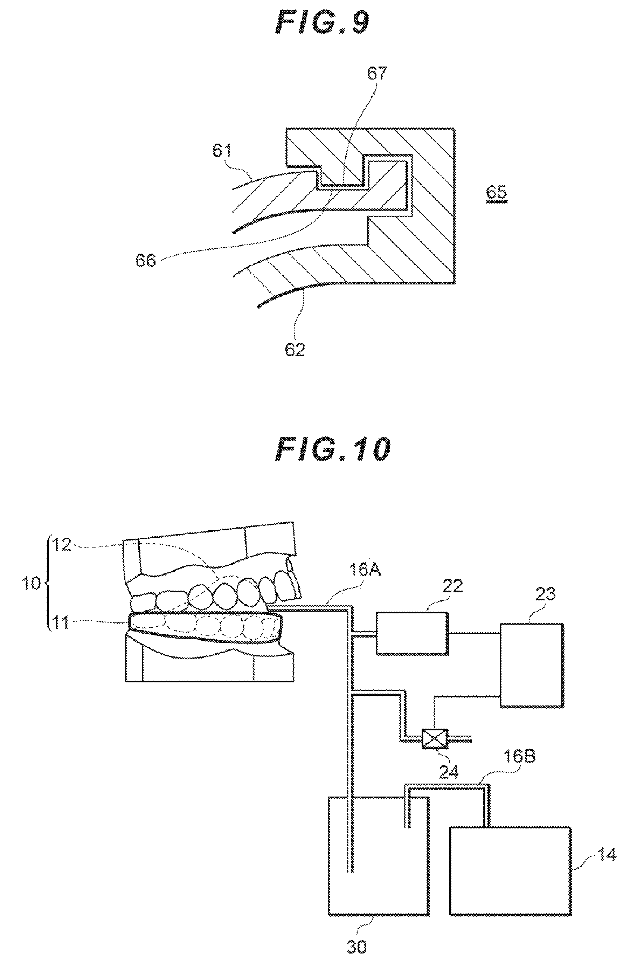

As shown in FIG. 8, the layer 62 on the side that contacts the tongue of the capsule 12 having a bilayer structure may also employ a removable configuration. In this case, the layers 61 and 62 can be tightly connected by, for example, a connecting portion 65 composed of an indentation 66 provided in the layer 61 and a projection 67 provided in an inner curved portion 62A of the layer 62 provided so as to engage therewith. FIG. 9 is a cross-sectional view of the connecting portion 65 taken along line IX-IX in FIG 6B. Furthermore, an outer curved portion 62B of the layer 62 is pressed against the upper surface of the layer 61 or the splint 11 by connecting the layer 61 and the layer 62 with the connecting portion 65, thereby enabling the formation of a dosed space between the layer 61 and the layer 62.

As a result of employing this configuration, since negative pressure can be nearly uniformly generated over a wide area of the tongue by means of the plurality of fine through holes 64 by suctioning air within the space between both layers through the fine through holes 64, the tongue can be held sufficiently. In addition, since the negative pressure is applied while dispersing, there is less likelihood of the occurrence of adverse side effects attributable to local excessive suction.

The size, shape, interval and so forth of the fine through holes 64 can be suitably changed provided the objective thereof is fulfilled.

The pressure generating device 14 that generates negative pressure used in the present invention may be any such device provided it is able to maintain the inside of the capsule 12 at negative pressure, examples of which include a suction pump or vacuum pump enabling adjustment of pressure and flow rate. A manual pump may also be used.

The tube 16 used in the present invention connects the through hole 13 of the capsule 12 and the pressure generating device 14, and for example, a catheter tube can be used for this tube 16. The tube 16 is removably attached to the through hole 13 and employs a configuration in which it fits into the through hole 13 so as to completely seal it. For example, the tube 16 may be fabricated with an elastic material, and the diameter of the end on the side connected to the through hole 13 may be formed to be larger than other portions. According to such a configuration, the tube 16 can be connected to the through hole 13 by inserting into the through hole 13 by applying pressure thereto. Alternatively, the tube 16 having a larger diameter than the diameter of the through hole 13 may be used, and may be connected by inserting into the through hole 13 by applying pressure thereto. Furthermore, the position of the through hole 13 is preferably close to the splint 11 within a range that does not obstruct the lips when the tube 13 is attached.

The tongue position controller 1 according to the present invention is preferably provided with a pressure adjustment mechanism 20 that automatically adjusts negative pressure generated by the pressure generating device 14. The pressure adjustment mechanism 20 can be composed of, for example, a pressure sensor 22 installed to enable measurement of negative pressure within the tube 16, a valve 24 provided in a path 17 connecting the tube 16 to the outside air, and a control device 23 that controls opening and closing of the valve 24 according to a measured value of the pressure sensor 22 (see FIG. 1). Although a solenoid valve or motorized valve and the like can be used for the valve 24, a solenoid valve is preferable in consideration of its rapid response speed.

In the case a time-based change in pressure is measured with the pressure sensor 22 and the measured value is below a prescribed valve, the control device 23 increases pressure by opening the valve 24 to prevent excessive suction. On the other hand, in the case the measured value is equal to or greater than a prescribed value and suction has become inadequate, the control device 23 is able to reduce internal pressure by closing the valve 24. In addition, a configuration may be employed in which the pressure generating device 14 is also connected to the control device 23, and pressure information from the pressure sensor 22 may be fed back to the pressure generating device 14. As a result of employing such a configuration, when excessive negative pressure is applied beyond a set pressure, a system switch can be turned off automatically thereby preventing overheating or failure and the like of the pressure generating device 14.

The TRD of the prior art is a so-called open loop system, and is not provided with means for confirming whether negative pressure is actually being generated in a socket for holding the tongue, the degree of that negative pressure in the case of being generated, or whether or not there is a change in pressure over time. Moreover, when a problem has occurred in controlling the position of the tongue due to insufficient negative pressure, there is also no means for adding negative pressure or means for relieving negative pressure in the case of being excessive. However, according to the tongue position controller of the present invention, pressure within the capsule 12 can be finely adjusted (titrated) by the pressure adjustment mechanism 20. In particular, once the tongue is held within the capsule 12 equipped with a valve by suction, that state can be subsequently maintained by a retaining a weak negative pressure load. As a result, adverse side effects attributable to the application of suction beyond that which is required can be prevented and energy can be conserved.

Moreover, in the tongue position controller according to the present invention, as shown in FIG. 10, a gas-liquid separator 30 is preferably arranged between the capsule 12 and the pressure generating device 14. Although the tongue is held when negative pressure is applied by the pressure generating device, secretions inside the oral cavity (such as saliva) may be suctioned simultaneously, thereby having an effect on measurement accuracy of the pressure sensor 22 and operation of the pressure generating device 14. Consequently, a device resembling a gas-liquid separator incorporated within a dental unit is installed in the tube 16, and liquid accumulates in this portion of the tube 16. Suctioned materials are discharged into the gas-liquid separator 30 through a first tube 16A, while only gas flows to the pressure generating device 14 from the gaseous phase portion of the gas-liquid separator through a second tube 16B. Furthermore, although the pressure sensor 22 and the valve 24 are connected to the first tube 16A in FIG. 10, both are also preferably connected to the second tube 16B.

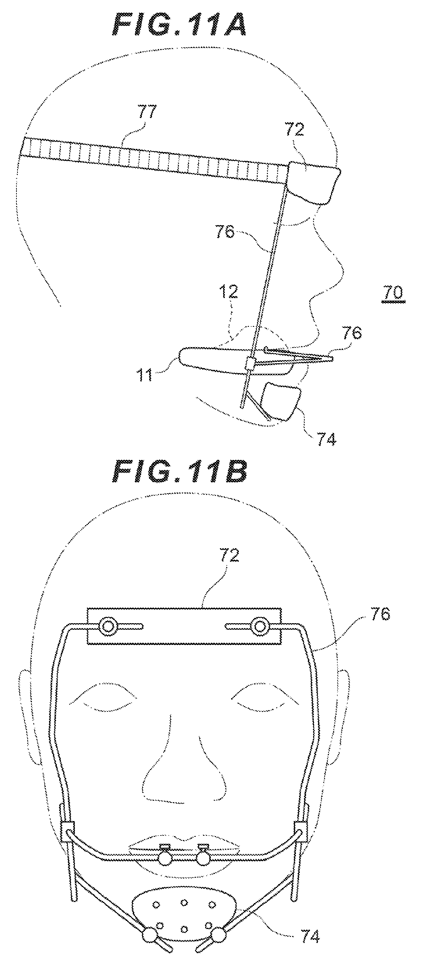

Moreover, as shown in FIG. 11, the tongue position controller according to the present invention preferably includes a face mask 70 that holds the mounting portion at a proper position. The face mask 70 is composed of a forehead retaining portion 72 that contacts the forehead, a lower jaw retaining portion 74 that contacts the lower jaw, a wire 76 that connects the forehead retaining portion 72, the lower jaw retaining portion 74 and the mounting portion 10, and a rubber headband 77 attached to the forehead retaining portion 72, and is fixed to the face by the rubber headband 77 as shown in the drawing.

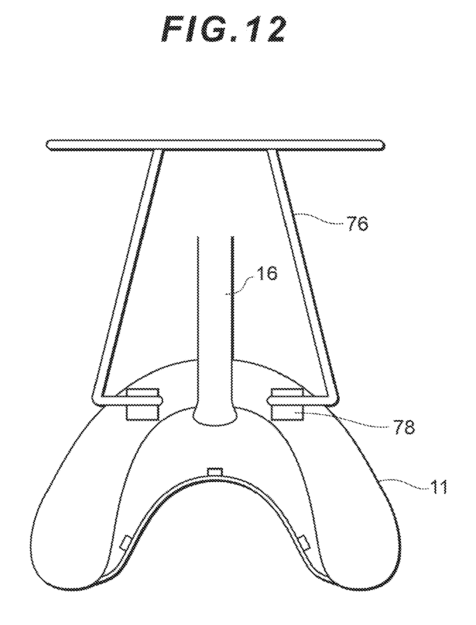

As shown in FIG. 12, the wire 76 removably engages with holes 78 provided in the lower jaw retaining portion 74.

As a result of using the face mask 70, the mounting portion 10 can be held at the proper position even for persons presenting with an edentulous jaw.

The tongue position controller 1 of the present invention is not limited to the previously described embodiments, but rather can be altered in shape or form in various ways within the scope of the gist of the present invention.

In addition, in the case of using the tongue position controller of the present invention, a substance that enhances adhesion to the tongue is preferably applied to the site of the capsule 12 that contacts the tongue. As a result, since tongue position can be maintained within the capsule 12, the burden of the negative pressure can be reduced and electric power can be conserved. For example, by applying a negative pressure load with a manual pump prior to going to bed, tongue position can be maintained within the capsule 12 throughout the night. Examples of such substances are substances having high consistency and low fluidity.

Next, an explanation is provided of the control method of the tongue position controller 1 according to the present invention. The control method of the tongue position controller 1 according to the present invention includes a step of comparing a measured value of a pressure sensor with a preset pressure (threshold value), and a step of opening a solenoid valve in the case the measured value has decreased to or below the threshold value. Here, the preset pressure (threshold value) can be determined according to the result of a method for evaluating therapeutic effect for OSAS to be subsequently described.

The control method of the tongue position controller 1 according to the present invention may further include a step of comparing a measured value of the pressure sensor with a preset threshold value when the solenoid valve is open, and a step of closing the solenoid valve in the case the measured value has increased to or above the preset threshold value. Negative pressure can be enhanced and suction can be increased by closing the solenoid valve. Repeatedly opening and closing the solenoid valve makes it possible to maintain a constant negative pressure applied to the tongue.

Determination of the threshold value of the tongue position controller 1 according to the present invention is carried out by obtaining an overnight polysomograph. Determination of this threshold value is similar to techniques routinely carried out when determining the optimum positive pressure threshold value (optimum pressure) for patients using nCPAP, and only differs with respect to the use of the tongue position controller 1 instead of nCPAP. In overnight in-hospital testing conducted at a specialized sleep center, electroencephalogram, respiration and pressure information from the pressure sensor 22 in the tongue position controller 1 were monitored and recorded in OSAS patients while using the tongue position controller 1. Setting of negative pressure (negative pressure titration) was carried out manually by a sleep technologist while observing a monitor. Negative pressure generated by the pressure generating device was set to a low level during sleep so as not to allow the patients' sleep to be impaired by excessive negative pressure loading. After the patients feel asleep, the occurrence of respiratory events (snoring, apnea, hypopnea, etc.) that changes according to sleep stage was observed while monitoring the patients, and negative pressure values were changed manually so as to eliminate these events. Finally, the negative pressure value at which these events were able to be eliminated as much as possible was defined as the "optimum pressure" that is equal to the threshold value. According to this technique, the optimum threshold value corresponding to each patient is determined, and this value is set for the tongue position controller. In the case of being provided with a pressure adjustment mechanism, the threshold value is stored in the pressure adjustment mechanism. Once the threshold value has been stored in the pressure adjustment mechanism, the position of the tongue is maintained and obstruction of the upper airway posterior to the soft palate can be effectively prevented by applying the mounting portion before going to sleep and sleeping white allowing the tongue position controller to demonstrate its action,

Although a patient can routinely improve OSAS and maintain the tongue in a favorably controlled state by using the tongue position controller 1 set to the threshold value determined by negative pressure titration as previously described, evaluation of the efficacy of the tongue position controller 1 according to the present invention is preferably carried out at the stage the patient has become familiar with use of the controller by focusing on the change in apnea-hypopnea index as determined by polysomnography, Moreover, evaluation of improvement of upper airway obstruction by the tongue position controller 1 according to the present invention is preferably carried out on the basis of both of morphology and physiological function. In order to evaluate changes in upper airway obstruction morphologically, lateral x-rays are taken of the head when the tongue position controller 1 is used, the sizes, positions and the like of the tongue, soft palate, mandible, maxilla and hyoid bone are evaluated, and the positional relationship of the upper airway with each tissue along with the anterior and posterior diameter of the upper airway are analyzed. In addition, information about the muscle fiber diffusion of the genioglossus muscle can also be obtained using Diffusion Tensor Magnetic Resonance Imaging (DTMRI) (Gilbert RJ, et al. Dysphagia 2005; 20:1-7). Moreover, the resistance of the nasal cavity and upper airway region can be measured while at rest during nose breathing while using the tongue position controller 1 for an evaluation of physiological function (Okawara Y, et al. Am J Orthod Dentfac Orthop 2004:126:620-622).

## Claims

1. A tongue position controller, comprising:
a mounting portion composed of a splint applied to a lower jaw dentition, and a capsule which is arranged on an inner curved portion of an upper surface of the splint, which is composed so as to cover at least a portion of a tongue when the splint is applied to the lower jaw dentition, and which has a through hole positioned at a tip of the tongue when the splint is applied;
a pressure generating device that generates a negative pressure; and
a tube that connects the through hole and the pressure generating device.

2. The tongue position controller according to claim 1, further comprising a pressure adjustment mechanism that automatically adjusts negative pressure generated by the pressure generating device.

3. The tongue position controller according to claim 2, wherein the pressure adjustment mechanism includes a pressure sensor that measures negative pressure in the tube, and a valve which is provided in a path connecting the tube with outside air, and an opening and closing of which is controlled according to a measured value of the pressure sensor.

4. The tongue position controller according to any one of claims 1 to 3, wherein the splint and the capsule are formed from a thermoplastic or thermosetting dental material.

5. The tongue position controller according to any one of claims 1 to 5, wherein the capsule has a bilayer structure in which a periphery is dosed, a plurality of fine through holes are provided in one layer that contacts the tongue, and a through hole to which the tube is connected is provided in the other layer.

6. The tongue position controller according to claim 5, wherein each layer of the bilayer structure employs a removable configuration.

7. The tongue position controller according to any one of claims 1 to 6, wherein a gas-liquid separator is arranged between the through hole of the capsule and the pressure generating device, and
the tube is composed of a first tube that connects the through hole of the capsule and the gas-liquid separator for discharging suctioned material inside the gas-liquid separator, and a second tube that connects a gaseous phase portion of the gas-liquid separator and the pressure generating device.

8. The tongue position controller according to any one of claims 1 to 7, further comprising a face mask which holds the mounting portion, and which includes a forehead retaining portion that contacts a forehead, a lower jaw retaining portion that contacts a lower jaw, and a wire that connects and immobilizes the forehead retaining portion, the lower jaw retaining portion and the mounting portion.

9. A control method of the tongue position controller according to claim 3, which comprises the step of:
opening the valve in a case that the measured value has decreased to or below the preset pressure.

10. The control method according to claim 9, which further comprises the steps of:
comparing a measured value of the pressure sensor with a preset pressure with the valve open; and
closing the valve in a case that the measured value has increased to or above the preset pressure,
